# EUROPEAN PATENT APPLICATION

(11) **EP 4 356 930 A1**
(43) Date of publication of application: **24.04.2024**
(21) Application number: 22203125.4
(22) Date of filing: 21.10.2022
(51) Int. Cl.: A61K 47/69, A61K 41/00

(54) **PHOTOSENSITIVE DIAZIRINE-CONJUGATED PHOSPHOLIPIDS AND USES THEREOF**

(71) Applicant: UNIVERSITE DE BORDEAUX, 33000 Bordeaux (FR); Institut Polytechnique de Bordeaux, 33400 Talence (FR); Centre National de la Recherche Scientifique, 75794 Paris (FR); INSTITUT NATIONAL DE LA SANTE ET DE LA RECHERCHE MEDICALE - INSERM, 75013 Paris (FR)
(72) Inventor: BADARAU, Edouard, 33140 VILLENAVE D ORNON (FR); BESTEL, ISABELLE, 33700 Mérignac (FR); ALIES, BRUNO, 33600 PESSAC (FR); SARYEDDINE, LILIAN, 33600 PESSAC (FR)
(74) Representative: Lecca, Patricia S.

(57) **Abstract**

The present invention relates to novel photosensitive diazirine-conjugated phospholipids, and their use in delivering and releasing an active ingredient in a controlled manner, as well as photosensitive liposomes and controlled delivery systems comprising one or more photosensitive diazirine-conjugated phospholipids. The invention also relates to compositions and kits comprising said photosensitive liposomes, for use in various fields, such as for example in therapy, diagnosis, cosmetics, etc...

## Description

### FIELD OF THE INVENTION

The present invention relates to novel photosensitive diazirine-conjugated phospholipids, and their use in delivering and releasing an active ingredient in a controlled manner, as well as photosensitive liposomes and controlled delivery systems comprising one or more photosensitive diazirine-conjugated phospholipids. The invention also relates to compositions, in particular pharmaceutical compositions comprising said photosensitive liposomes and uses thereof.

### BACKGROUND OF THE INVENTION

The use of drug delivery systems providing both slow release of drugs and specific targeting of drugs to the desired/affected organs/systems to reduce systemic distribution and exposure of non-target organs has long been sought. Over the past several decades, various photosensitive drug delivery formulations like nanoparticles have been developed as potential therapies, ranging from photodynamic therapy technologies that have reached clinical use, to delivery systems that are still in the early clinical stages. Many of these systems are designed to achieve on-demand drug release via photo-triggerable mechanisms. Combinations of multiple treatment modalities using photosensitive delivery systems have also gathered great interest in combating multi-drug resistant cancers due to their synergistic effects. One of the promising candidates that has been developed in this field includes the phospholipid (PL) liposomes and their derivatives.

Liposomes are small, spherical vesicles composed of phospholipids and consist of one or more lipid bilayers enclosing an aqueous core. Liposomes can encapsulate both hydrophilic drugs (in the aqueous interior) and lipophilic drugs (in the lipid bilayer) and are thus highly suitable for drug delivery. Phospholipids are amphiphilic lipid molecules found in living tissues and comprise one of the predominant components of cell membranes, a self-assembling bilayer structure. The ability of some phospholipids to assemble into liposomes, *i.e*., enclosed membrane structures, either alone or in the presence of cholesterol, makes them suitable for a wide array of potential applications including their use as a simplified artificial cell-model in biology-related research, nano- and micro-reactors, bio-imaging vehicles, and drug delivery systems (DDS).

Phospholipid liposomes can serve as sustained-release or controlled-release drug delivery systems, thus contributing to improvement in drug efficacy and allowing reduction in the frequency of dosing. By providing protection of both the entrapped drug and the biological environment, liposomes reduce the risks of drug inactivation and drug degradation. Since the pharmacokinetics of free drug release from the particles are different from directly administered free drug, these carriers can be used to reduce toxicity and undesirable side effects.

Natural phospholipids are not photo-responsive, so photo-responsive functionalities are often introduced into molecular structures to prepare photo-responsive liposomes. We can cite as example a phosphotidyl choline lipid which comprises an O-nitrobenzyl-structure beside the glycerol-linker, and liposomes formed by this functionalized phospholipid exhibit significant photo-responsiveness.

It is important to note that liposomes are not only used in oncology, but also commonly used to treat and/or prevent fungal infections (Amphotericin B as PA in Abelet, Amphotec or Ambisome) and in vaccines such as hepatitis A (Epaxal), influenza (Inflexal), and Malaria (Mosquirix). Besides therapeutic applications, liposomes can also be used in the food industry, cosmetics, as well as in medical alternostics.

Some approaches have been developed using the chemically reactive group of natural phospholipids to introduce photo-responsive functionalities in liposomes, such as the primary amine head group of phosphotidyl ethanolamine. In addition to the difficulties in purification of zwitterionic molecules in these approaches, such photo-responsive phospholipids are unable to assemble into vesicular structure. This issue often necessitates inclusion of other natural phospholipids to form a liposome matrix in which the photo-responsive phospholipids are inserted. Unfortunately, addition of matrix-phospholipids can lead to a change in response kinetics and extent. In the research of photo-responsive DDS, liposomes with excellent photo-responsive kinetics and extent are important in both fundamental studies and in application-oriented research. In addition to the spatial and temporal control afforded by irradiation, the light exposure provides a non-invasive trigger to induce property changes in liposomes such as a phase-transition in the bilayer membrane, permeability enhancements and shape changes. Practically, a rapid photo-responsiveness enhances the spatial and temporal control associated with the use of light to facilitate enhanced response in DDS. Thus, an ideal photo-responsive liposome-based DDS should be composed of phospholipids which are chemically modified to have rapid photo-responsiveness while preserving the ability to self-assemble into liposomes, rather than embedding photo-responsive molecules into the matrix liposome.

US Patent no. 8,535,712 describes a more rigid membrane obtained by crosslinking a small amount of photo-polymerizable lipids in order to increase permeability to drug like molecules entrapped in the liposome. However, this system discloses the use of photo-polymerizable lipids that are distributed evenly throughout the liposome membrane, thus limiting the impact of the induced crosslinking. Accordingly, there is still a need for a liposome delivery vehicle that has sufficient stability to allow for effective delivery to a target site while having a triggering mechanism that effectively releases the liposomal content at the target site.

The Applicants have developed a unique phospholipid using a diazirine derivative as a photoreactive group. Diazirines are stable heterocyclic groups, containing an azo group and a sp3 carbon. Diazirine has the advantage of favoring the irreversible binding compared to other photoreactive groups. Therefore, the present invention provides photosensitive diazirine-conjugated phospholipids which are capable of forming self-assemblies with increased stability compared to conventional phospholipids.

Also provided herein is a method for producing the same and use thereof in controlling the release of one or more active agents, wherein the said method includes a step of exposing said liposome to direct UV irradiation and/ or to UV-generating radiation sources, which allows photo-induced modification of the said diazirine-conjugated phospholipids and results in the release of said one or more active ingredients from the liposome.

### SUMMARY OF THE INVENTION

The present invention provides novel photosensitive diazirine-conjugated phospholipids (photosensitive-PL) defined herein below by their following general formula. Modification of these novel phospholipid derivatives may be photo-induced, and thus are particularly useful for the preparation of photosensitive liposomes for controlled delivery of active agents encapsulated therein. The present invention also provides photosensitive liposomes which comprise one or more photosensitive diazirine-conjugated phospholipids. Such photosensitive liposomes advantageously comprise one or more active agents encapsulated within said liposome, which may be delivered upon photo-activation of the said diazirine-conjugated phospholipids constitutive of said photosensitive liposomes.

The present invention further provides a photosensitive liposomal controlled delivery system, comprising photosensitive liposomes as described above, enabling the release of said one or more active agents in a controlled manner by an exposure to direct UV irradiation, or by the UV-generated radiation sources.

These photosensitive liposomes and/or photosensitive liposomal controlled delivery systems are useful for controlling the release of said one or more active agents, comprising exposing said liposome to direct UV irradiation, to UV-generating radiation sources, thereby allowing photo-induced modification of the said diazirine-conjugated phospholipids and releasing of said one or more active ingredients from the liposome.

The present invention finally provides intermediates useful for in the preparation of photosensitive diazirine-conjugated phospholipids.

### BRIEF DESCRIPTION OF THE FIGURES

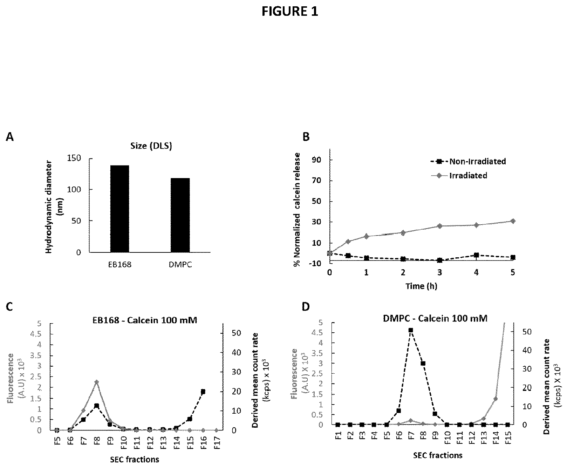

**Figure 1****:** (A) size comparison (by DLS) between liposomes obtained from photosensitive phospholipids EB168 (See Example 3) *versus* liposomes obtained from the same phospholipids devoid of the photosensitive moiety (DMPC); (B) fluorescence results for the normalized calcein release from irradiated *versus* non-irradiated liposomes obtained from EB168; (C) fluorescence and derived mean count rate (by DLS) profiles of different size-exclusion chromatography (SEC) fractions obtained during the separation of calcein-loaded EB 168-based liposomes from the free calcein; (D) fluorescence and derived mean count rate (DLS) profiles of different size-exclusion chromatography (SEC) fractions obtained during the separation of calcein-loaded DMPC-based liposomes from the free calcein.

### DETAILED DESCRIPTION

The present invention thus provides novel photosensitive diazirine-conjugated phospholipids (photosensitive-PL) having the following general formula (I): 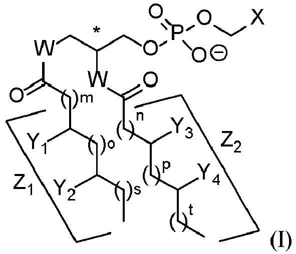
wherein the radical X is chosen among the following groups: 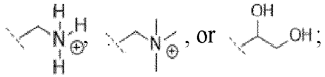
wherein at least one radical Y₁, Y₂, Y₃ or Y₄ is a diazirine group: 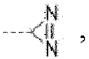 the other radical Y₁, Y₂, Y₃ or Y₄ being a hydrogen;
wherein the atom W is O or N;
wherein said phospholipids are saturated or unsaturated fatty chains Z₁ or Z₂;
wherein m, n, o, p, s, t are integers independently equal to 1 to 8; or 1 to 7, or 1 to 6, or 1 to 5; and
wherein said phospholipid is either achiral or chiral as defined by the absolute configuration of the stereogenic center C^{∗} (R, S or a racemic mixture). Salt derivatives thereof are also encompassed.

Preferred photosensitive diazirine-conjugated phospholipids (photosensitive-PL) comprise phospholipids having the general formula (I) wherein W=O. These photosensitive-PL thus have the following general formula (II): 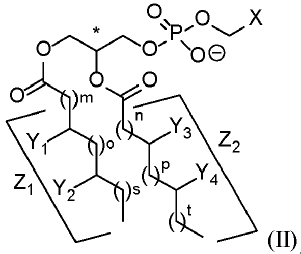
wherein the radical X is chosen among the following groups: 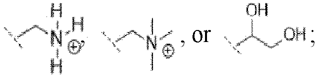
wherein at least one radical Y₁, Y₂, Y₃ or Y₄ is a diazirine group: 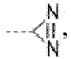 the other radical Y₁, Y₂, Y₃ or Y₄ being a hydrogen;
wherein said phospholipids are saturated or unsaturated fatty chains Z₁ or Z₂;
wherein m, n, o, p, s, t are integers independently equal to 1 to 8, or 1 to 7, or 1 to 6, or 1 to 5; and wherein said phospholipid is either achiral or chiral as defined by the absolute configuration of the stereogenic center C^{∗} (R, S or a racemic mixture);
and/or salts thereof.

Most preferred are photosensitive diazirine-conjugated phospholipids of formula (I) or (II) above wherein t is an integer independently equal to 1 to 6 and to 8.

By way of example, various phospholipids incorporating the diazirine moiety within the above general formulas (I) or (II) have been listed in the Table 1 below:

**Table 1:**

| No | Structure of phospholipid incorporating the diazirine moiety | Name |
|---|---|---|
| 1 | 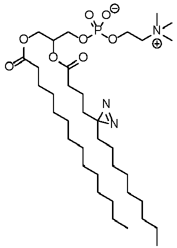 | 2-((4-(3-nonyl-3H-diazirin-3-yl)butanoyl)oxy)-3-(tetradecanoyloxy)propyl (2-(trimethylammonio)ethyl) phosphate |
| 2 | 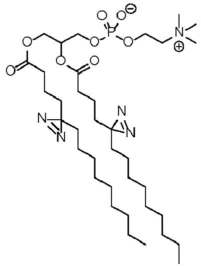 | 2,3-bis((4-(3-nonyl-3H-diazirin-3-yl)butanoyl)oxy)propyl (2-(trimethylammonio)ethyl) phosphate |
| 3 | 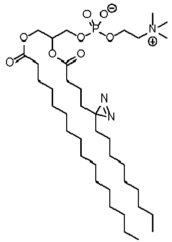 | 2-((4-(3-nonyl-3H-diazirin-3-yl)butanoyl)oxy)-3-(palmitoyloxy)propyl (2-(trimethylammonio)ethyl) phosphate |
| 4 | 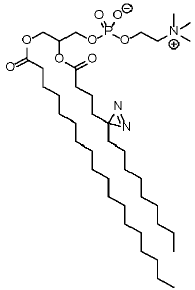 | 2-((4-(3-nonyl-3H-diazirin-3-yl)butanoyl)oxy)-3-(stearoyloxy)propyl (2-(trimethylammonio)ethyl) phosphate |
| 5 | 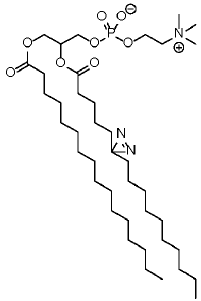 | 2-((5-(3-decyl-3H-diazirin-3-yl)pentanoyl)oxy)-3-(palmitoyloxy)propyl (2-(trimethylammonio)ethyl) phosphate |
| 6 | 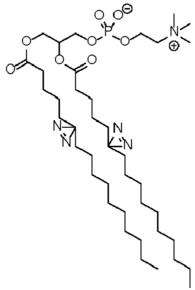 | 2,3-bis((5-(3-decyl-3H-diazirin-3-yl)pentanoyl)oxy)propyl (2-(trimethylammonio)ethyl) phosphate |
| 7 | 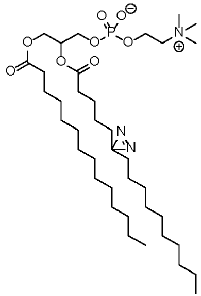 | 2-((5-(3-decyl-3H-diazirin-3-yl)pentanoyl)oxy)-3-(tetradecanoyloxy) propyl (2-(trimethylammonio)ethyl) phosphate |
| 8 | 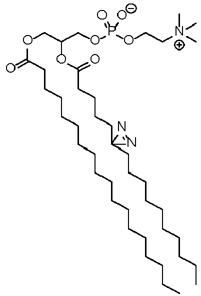 | 2-((5-(3-decyl-3H-diazirin-3-yl)pentanoyl)oxy)-3-(stearoyloxy)propyl (2-(trimethylammonio)ethyl) phosphate |
| 9 | 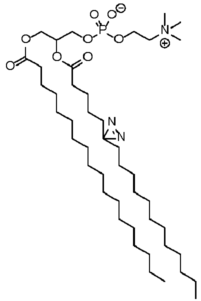 | 2-((5-(3-dodecyl-3H-diazirin-3-yl)pentanoyl)oxy)-3-(stearoyloxy)propyl (2-(trimethylammonio)ethyl) phosphate |
| 10 | 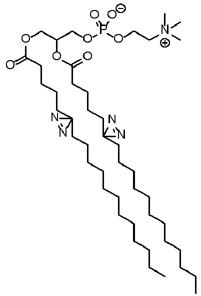 | 2,3 -bis((5 -(3 -dodecyl-3H-diazirin-3 - yl)pentanoyl)oxy)propyl (2-(trimethylammonio)ethyl) phosphate |
| 11 | 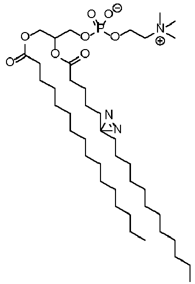 | 2-((5-(3-dodecyl-3H-diazirin-3-yl)pentanoyl)oxy)-3-(palmitoyloxy)propyl(2-(trimethylammonio)ethyl) phosphate |
| 12 | 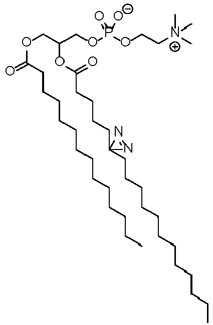 | 2-((5-(3-dodecyl-3H-diazirin-3-yl)pentanoyl)oxy)-3-(tetradecanoyloxy)propyl (2-(trimethylammonio)ethyl) phosphate |
| 13 | 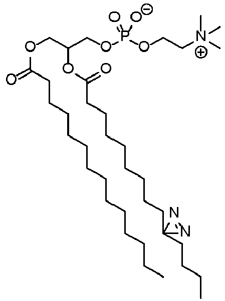 | 2-((9-(3-butyl-3H-diazirin-3-yl)nonanoyl)oxy)-3-(tetradecanoyloxy)propyl (2-(trimethylammonio)ethyl) phosphate |
| 14 | 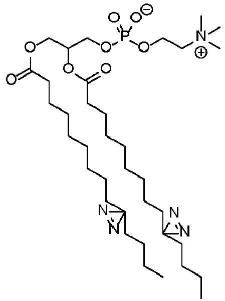 | 2,3-bis((9-(3-butyl-3H-diazirin-3-yl)nonanoyl)oxy)propyl (2-(trimethylammonio)ethyl) phosphate |
| 15 | 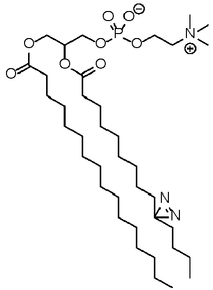 | 2-((9-(3-butyl-3H-diazirin-3-yl)nonanoyl)oxy)-3-(palmitoyloxy) propyl (2-(trimethylammonio)ethyl) phosphate |
| 16 | 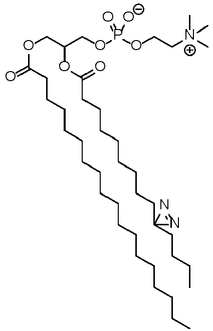 | 2-((9-(3-butyl-3H-diazirin-3-yl)nonanoyl)oxy)-3-(stearoyloxy)propyl (2-(trimethylammonio)ethyl) phosphate |
| 17 | 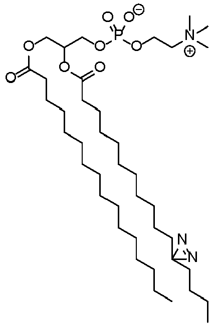 | 2-((11-(3-butyl-3H-diazirin-3-yl)undecanoyl)oxy)-3-(palmitoyloxy)propyl (2-(trimethylammonio)ethyl) phosphate |
| 18 | 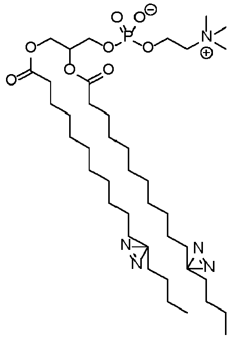 | 2,3-bis((11-(3-butyl-3H-diazirin-3-yl)undecanoyl)oxy)propyl (2-(trimethylammonio)ethyl) phosphate |
| 19 | 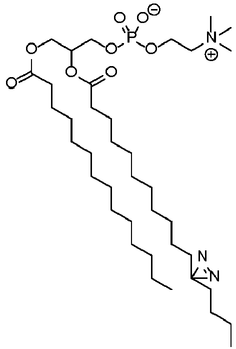 | 2-((11-(3-butyl-3H-diazirin-3-yl)undecanoyl)oxy)-3-(tetradecanoyloxy)propyl (2-(trimethylammonio)ethyl) phosphate |
| 20 | 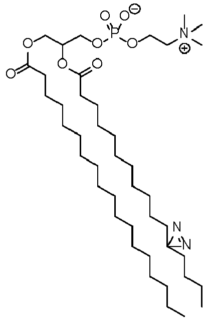 | 2-((11-(3-butyl-3H-diazirin-3-yl)undecanoyl)oxy)-3-(stearoyloxy)propyl (2-(trimethylammonio)ethyl) phosphate |
| 21 | 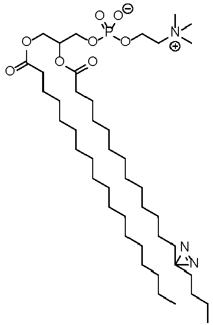 | 2-((13-(3-butyl-3H-diazirin-3-yl)tridecanoyl)oxy)-3-(stearoyloxy)propyl (2-(trimethylammonio)ethyl) phosphate |
| 22 | 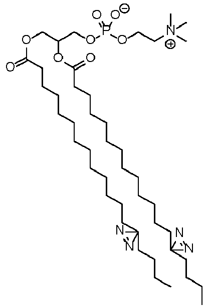 | 2,3-bis((13-(3-butyl-3H-diazirin-3-yl)tridecanoyl)oxy)propyl (2-(trimethylammonio)ethyl) phosphate |
| 23 | 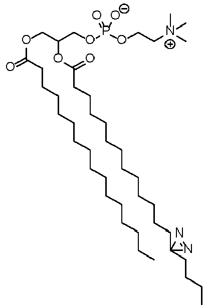 | 2-((13-(3-butyl-3H-diazirin-3-yl)tridecanoyl)oxy)-3-(palmitoyloxy)propyl (2-(trimethylammonio)ethyl) phosphate |
| 24 | 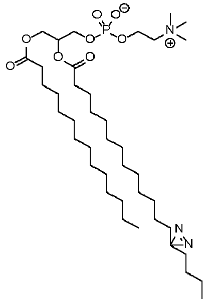 | 2-((13-(3-butyl-3H-diazirin-3-yl)tridecanoyl)oxy)-3-(tetradecanoyloxy)propyl (2-(trimethylammonio)ethyl) phosphate |
| 25 | 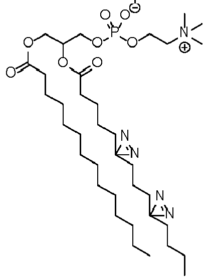 | 2-((5-(3-(3-(3-butyl-3H-diazirin-3-yl)propyl)-3H-diazirin-3-yl)pentanoyl)oxy)-3-(tetradecanoyloxy)propyl (2-(trimethylammonio)ethyl) phosphate |
| 26 | 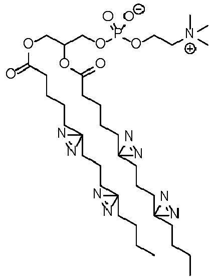 | 2,3-bis((5-(3-(3-(3-butyl-3H-diazirin-3-yl)propyl)-3H-diazirin-3-yl)pentanoyl)oxy)propyl (2-(trimethylammonio)ethyl) phosphate |
| 27 | 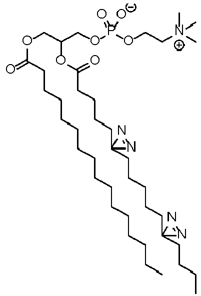 | 2-((5-(3-(5-(3-butyl-3H-diazirin-3-yl)pentyl)-3H-diazirin-3-yl)pentanoyl)oxy)-3-(palmitoyloxy)propyl (2-(trimethylammonio)ethyl) phosphate |
| 28 | 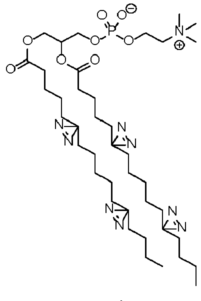 | 2,3-bis((5-(3-(5-(3-butyl-3H-diazirin-3-yl)pentyl)-3H-diazirin-3-yl)pentanoyl)oxy)propyl (2-(trimethylammonio)ethyl) phosphate |
| 29 | 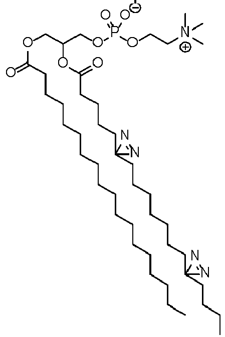 | 2-((5-(3-(7-(3-butyl-3H-diazirin-3-yl)heptyl)-3H-diazirin-3-yl)pentanoyl)oxy)-3-(stearoyloxy)propyl (2-(trimethylammonio)ethyl) phosphate |
| 30 | 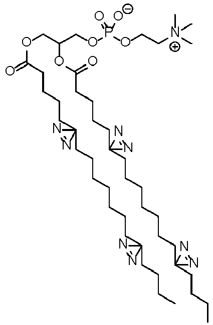 | 2,3-bis((5-(3-(7-(3-butyl-3H-diazirin-3-yl)heptyl)-3H-diazirin-3-yl)pentanoyl)oxy)propyl (2-(trimethylammonio)ethyl) phosphate |

Examples of diazirine-conjugated phospholipids of general formula (II) according to the present invention may be compound having the formula (X) below: 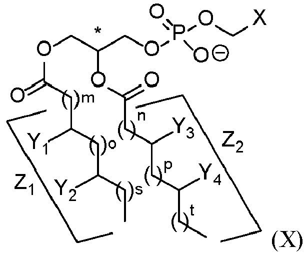
wherein m, n, o, p, s, t are integers independently equal to 1 to 8; or 1 to 7, or 1 to 6, or 1 to 5; and
wherein radicals X, Y₁, Y₂, Y₃ and Y₄ are defined in the Table 2 below:

**Table 2:**

| X | Y1 | Y2 | Y3 | Y4 |
|---|---|---|---|---|
| 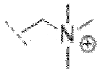 | H | H | H | 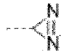 |
| 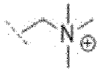 | H | H | 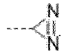 | H |
| 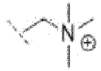 | H | 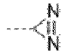 | H | H |
| 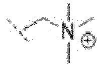 | 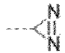 | H | H | H |
| 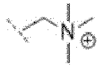 | H | 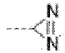 | H | 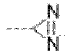 |
| 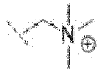 | 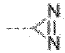 | H | 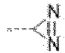 | H |
| 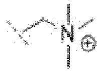 | 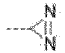 | H | H | 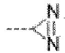 |
| 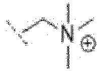 | H | 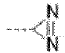 | 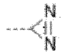 | H |
| 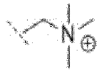 | 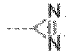 | 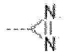 | 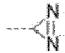 | 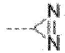 |
| 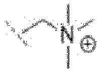 | H | H | 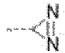 | 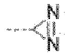 |
| 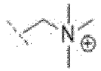 | 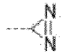 | 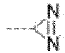 | H | H |

Photosensitive diazirine-conjugated phospholipids (photosensitive-PL) according to the present invention may thus be, without any limitations:
- 2-(4-(3-nonyl-3H-diazirin-3-yl)butanoyloxy)-3-(tetradecanoyloxy)propyl 2-(trimethyl ammonio) ethyl phosphate and has the following formula (III): 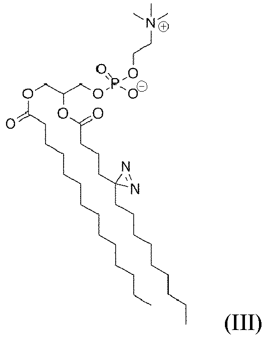
- 2-(4-(3-nonyl-3H-diazirin-3-yl)butanoyloxy)-3-(pentadecanoyloxy)propyl 2-(trimethyl ammonio)ethyl phosphate of formula (IV) below: 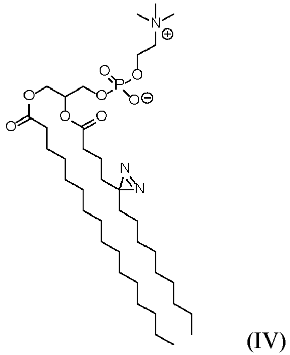
- 2-(4-(3-nonyl-3H-diazirin-3-yl)butanoyloxy)-3-(stearoyloxy)propyl 2-(trimethyl ammonio) ethyl phosphate:

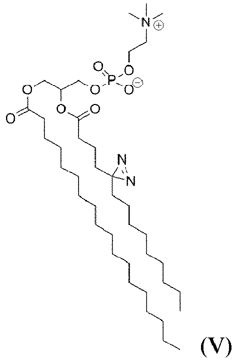

Modification of the diazirine group in the photosensitive-PL is photo-induced. Indeed, the photosensitive diazirine group is a functional group which may be photo-activated and thus undergoes structural changes upon exposure to direct UV irradiation, or to UV-generated radiation sources, thereby allowing formation of a covalent bond between the diazirine group and a substituent of another compound.

UV-generating radiation sources may be chosen among scintillators and/or up-conversion materials which generate UV when exposed to X-ray, and/or near-infrared (NIR) light, respectively. The scintillators and/or upconversion materials which generate UV when exposed to X-ray, and/or near-infrared (NIR) light may be for example nanomaterials or lanthanides chosen among lanthanum, cerium, ytterbium, yttrium, erbium, thulium, and/or salts thereof.

X-ray may be generally obtained from a source electromagnetic energy having a wavelength of less than 100 angstroms. In the same manner, NIR light may be obtained from a source of electromagnetic energy having a wavelength approximately 700 nm to 1400nm.

Photosensitive diazirine-conjugated phospholipids according to the present invention may comprise either natural or synthetic phospholipids. Natural phospholipids are defined as phospholipids isolated from natural sources like, *e.g.*, soybean, rapeseed, and sunflower seed. Natural phospholipids may be further converted to saturated phospholipids by means of hydrogenation or further treated with enzymes to, *e.g*., remove partially fatty acids or to convert a polar head group. The saturated phospholipids are considered as natural phospholipids because the resulting saturated lipids are also occurring in nature (*i.e.*, natural identical). Typical for all natural phospholipids is that the eventual fatty acid and polar head group composition is determined by the (phospholipid and fatty acid) composition of the starting natural material and applied isolation procedures. In contrast, synthetic phospholipid are phospholipids where specific molecular species of polar head groups or fatty acids are introduced by means of a tailor-made chemical synthesis process.

Preferred photosensitive diazirine-conjugated phospholipids may be chosen among phosphatidylcholine, phosphatidylserine, phosphatidylinositol, phosphatidic acid, phosphatidylglycerol, or phosphatidylethanolamine.

According to a second aspect, the present invention is directed to photosensitive liposomes comprising one or more photosensitive diazirine-conjugated phospholipids of formula (I) or (II) as described above. Preferred photosensitive liposomes comprise photosensitive diazirine-conjugated phospholipids of formula (II) as described above.

Photosensitive liposomes according to the present invention may be standard vesicular self-assemblies composed of phospholipids which are capable of inducing photochemical reactions under suitable conditions to form the liposome bilayers, with the particularity that they comprise one or more photosensitive diazirine-conjugated phospholipids of formula (I) or (II).

Photosensitive liposomes according to the present invention are particular useful as carriers, since due to their vesicular structures, they can encapsulate substances with different solubility. They have a size comprised between 100-200 nm. Photosensitive liposomes are non-toxic, biocompatible and biodegradable and the use of phospholipid vesicles contributes to reducing toxicity and improving the therapeutic index of the loaded cargo. Additionally, liposomes show increased efficacy due to their effective protection of the loaded cargo against enzymatic degradation, as well as sustained and site-specific delivery.

Therefore, according to a preferred embodiment, these photosensitive liposomes comprise one or more active substances or agents encapsulated within said liposomes. Encapsulated substances may be hydrophilic compounds which possess an affinity to the aqueous space, which is created by the phospholipid bilayer. Such hydrophilic compounds may be active ingredients with potential in the medical field, cosmetic and/or pharmaceutical industries, which may be thus release in a controlled manner upon UV exposure of the liposomes, as described above. The active agents may be encapsulated within the photosensitive liposomes by either embedding in the lipid bilayer or locating in the aqueous lumen of the liposome.

Exposure of said photosensitive liposomes to direct UV irradiation, or to UV-generated radiation sources allows activating the photosensitive diazirine group present on said conjugated phospholipids within the liposomes, resulting in (1) crosslinking of the membrane components of the lipid bilayer and/or (2) generation of photo-oxidation/photo-reduced products from the photo-sensitive phospholipids, and thus in the controlled release of the liposome contents.

UV generating radiation sources may be chosen among scintillators and/or upconversion materials which generate UV when exposed to X-ray, and/or near-infrared (NIR) light, respectively. The scintillators and/or up-conversion materials which generate UV when exposed to X-ray, and/or near-infrared (NIR) light are nanomaterials or lanthanides chosen among lanthanum, cerium, ytterbium, yttrium, erbium, thulium, and/or salts thereof. Alternatively, said photosensitive liposomes may also comprise such scintillator that is responsive to radiation. Such scintillator may be exposed to radiation, such as X-rays, to cause the scintillators to emit UV light.

In addition to said one or more photosensitive diazirine-conjugated phospholipids of formula I, photosensitive liposomes may optionally comprise non-diazirine conjugated phospholipids. The proportion of diazirine-conjugated phospholipids within said photosensitive liposomes may be between 5-100%.

Structurally, these liposomes are microscopic vesicles comprising lipid bilayer(s) with variable sizes and shapes from long tubes to spheres, and from 25 nm and as large as 500 nm in diameter. Preferably, they are small single lamellar vesicles having a diameter from 50 to 250nm, or around 100 to 200nm. They may comprise natural phospholipids such as phosphatidylcholine (PC), phosphatidylethanolamine (PE), phosphatidic acid (PA), phosphatidylglycerol (PG), phospatidylserine (PS), and phosphatidylinositol (PI), as well as non-natural lipids such as for example distearoylPC; bis-SorbPC 17, 17; 1,2-distearoyl-sn-glycero-3-phosphocholine (DSPC); 1,2 dioleoyl-3-trimethylammonium-propane (DOTAP); dioctadecyldimethyl ammonium chloride (DODAc); 1,2-dimyristoyloxypropyl-3-dimethyl-hydroxyethylammonium (DMRIE); 2,3-dioleoyloxy-N-(2(sperminecarboxamide)ethyl)-N,N-dimethyl-1 propananninium (DOSPA); 1,2dimethyl-dioctadecylammoniumbromide (DDAB); 2-dioleyl-3-N,N,N-trimethylaminopropanechloride (DOTMA); 1,2-dimyristoy1-3-trimethylammonium propane (DMTAP); 1,2-distearoyl-3-trimethylammoniumpropane (DSTAP); 1,2-dioleoyl-3-dimethylammoniumpropane (DODAP); 1,2-dioleoyl-snglycero-3-phosphoethanolamine (DOPE); N-(4-carboxybenzyl)-N,N-dimethyl-2,3-bis(oleoyloxy)propan-1-aminium (DOBAQ); and dioctadecyl amidoglycylspermine (DOGS).

Such hydrophilic active agents may be chosen without any limitations among drugs, enzymes, peptides, proteins, toxins, nucleic acids, chemotherapeutics, pharmaceuticals, therapeutically active agents, antibodies, cosmetic agents, antifungal agents, diagnostic agents, cell toxins, radiopharmaceuticals, radiation sensitizers, contrast agents, iodinated agents, fluorescent compounds, and/or a combination thereof. Preferred drug encapsulated within photosensitive liposomes may be one or more anticancer drugs. Said anticancer drugs may be selected from, but not limited to, Cisplatin, Paclitaxel, Cytarabine, Docetaxel, Vincristine, Camptothecin, Topotecan, Oxaliplatin, Daunorubicin, Irinotecan, Doxorubicin, rapamycin, etc....

Diagnostic agents may be chosen among for example and without any limitations among x-ray imaging agent ethyl 3,5-diacetoamido-2,4,6-triiodobenzoate, the ethyl ester of diatrazoic acid and the like.

The photosensitive liposomes may accumulate in the target tissues either passively or can be targeted to the desired location using techniques known to those skilled in the art. In order to improve liposome targeting, said photosensitive liposome may also comprise a targeting molecule on the external liposomal surface. Such targeting molecules that can be used on the external liposomal surface may be for example endogenous molecules (nutriments, vitamins, hormones, neurotransmitters, etc...) required by cells survival and functioning, and/or synthetic analogues of such molecules designed to enhance their biological properties. Such modified photosensitive liposomes may for example effectively target the tumor site in a cancer patient.

Photosensitive liposome of the present invention may further comprise additional components, such as polyethylene glycol (PEG) or similar materials which are well-known to those skilled in the art to stabilize the liposome prior to UV exposure. PEGylated liposomes can not only prevent liposomes from fusing with one another but also enhance their *in vivo* circulation lifetime by suppressing plasma proteins from adsorbing onto the liposome.

According to a third aspect, the present invention provides compositions or kits comprising any of the photosensitive liposomes and encapsulated active agent as described above.

Said compositions or kits may be pharmaceutical compositions or kits which further comprise one or more therapeutically active agents and a pharmaceutically acceptable excipient, carrier, or diluent.

Alternatively, said compositions or kits may be diagnostic compositions or kits which thus further comprise one or more diagnostic agents and an acceptable excipient or carrier. The present invention also provides a method for performing diagnostic imaging in a subject, comprising the step of administering a diagnostic agent encapsulated in a liposome prepared according to a method of the present invention. Exemplary diagnostic agents include electron dense material, magnetic resonance imaging agents, radiopharmaceuticals, paramagnetism molecules, magnetic molecules, x-ray preparation and contrast agents, fluorescent chemicals/molecules, a nucleic acid, enzymes, a peptide or a small molecule. Radionucleotides useful for imaging include radioisotopes of copper, gallium, indium, rhenium, and technetium, including isotopes ⁶⁴Cu, ⁶⁷Cu, ¹¹¹In, ^{99m}Tc, ⁶⁷Ga or ⁶⁸Ga. According to another embodiment, said compositions or kits may be cosmetic compositions or kits and further comprise one or more cosmetic agents such as UV absorbers, after-sun treatment materials, occlusive oils, emollients, humectants, therapeutic and essential oils, perfumes, antiperspirants, moisturizers, color cosmetic materials, and even combinations of such materials.

As used herein the term "cosmetic" means an agent utilized, and/or intended to be applied to the human body for cleansing, beautifying, promoting attractiveness, altering the appearance of the skin, or any combination thereof. In a preferred embodiment the present invention provides compositions or kits comprising any of the photosensitive liposomes and encapsulated active agent specifically for cosmetic products. They can be used in classical formulations like suspensions, oil-in-water and water-in-oil emulsions and mixed micelles. These formulation types can be made or have particle sizes which lie in the range of nanoparticles.

Such cosmetic agent may be selected from the group consisting of vitamins, cosmetic peptides, oil control agents, sensation modifying agents, skin lightening agents, hydrating formulations, a compound that absorbs or reflects UV photons, other skin care agents, and combinations thereof.

In preferred the cosmetic agent is selected from the group consisting of 3-O-ethyl ascorbic acid, niacinamide, retinaldehyde hydroxysomes, retinyl palmitate, hydroxyapatite, whey protein, Pyrus Malus (apple) fruit extract, calcium PCA, magnesium PCA, retinal, sea salt, adenosine, ceramide NP, cholesterol, ceramide NS, ceramide AP, ceramide EOP, ceramide EOS, caprooyl phytosphingosine, caprooyl sphingosine, ascorbyl palmitate, tocopherol, glycoin, hyaluronic acid, hyaluronic acid derivative, and combinations thereof.

Cosmetic agent may be a vitamin selected from the group comprising of vitamin D, vitamin K, vitamin B (including niacinamide, nicotinic acid, Ci-is nicotinic acid esters, and nicotinyl alcohol; B6 compounds, such as pyroxidine; and B5 compounds, such as panthenol, or "pro-B5"), vitamin A (including retinoids such as retinyl propionate, carotenoids, and other compounds), vitamin E (including tocopherol sorbate, tocopherol acetate, other esters of tocopherol), vitamin C (including ascorbyl esters of fatty acids, and ascorbic acid derivatives, for example, ascorbyl glucoside, magnesium ascorbyl phosphate, sodium ascorbyl phosphate, and ascorbyl sorbate, and all natural and/or synthetic analogs thereof, and combinations thereof.

Cosmetic agent may also be chosen from peptides selected from the group consisting of di-, tri-, tetra-, penta-, and hexa-peptides, their salts, isomers, derivatives, and mixtures thereof. Examples of useful peptide derivatives include, but are not limited to, peptides derived from lentils and nuts like soy proteins, palmitoyl-lysine-threonine (pal- KT), palmitoyl-lysine-threonine-threonine-lysine-serine (MATRIXYL^{®}), palmitoyl-glycine- glutamine-proline-arginine (RIGIN^{®}), acetyl dipeptide-1 cetyl ester (IDEALIFT^{™}), and Cu- histidine-glycine-glycine (Cu-HGG, also known as IAMIN^{®}), and naturally occurring and synthesized derivatives thereof, and combinations thereof.

In certain other embodiments, the cosmetic agent is selected from oil control agents selected from the group consisting of compounds useful for regulating the production of skin oil, or sebum, and for improving the appearance of oily skin. Examples of oil control agents include, for example, salicylic acid, dehydroacetic acid, benzoyl peroxide, vitamin B3 (for example, niacinamide), and the like, their isomers, esters, salts and derivatives, and mixtures thereof.

Cosmetic agent may be selected from other skin care agents selected from the group consisting of retinol, steroids, salicylate, minocycline, antifungals, peptides, antibodies, lidocaine, and the like and combinations thereof. In some embodiments, other skin care agents include N-acyl amino acid compounds comprising, for example, N- acyl phenylalanine, N-acyl tyrosine, and the like, their isomers, comprising their D and L isomers, salts, derivatives, and mixtures thereof. An example of a suitable N-acyl amino acid is N-undecylenoyl-L-phenylalanine is commercially available under the tradename SEPIWHITE^{®}. Other skin active agents include, but are not limited to, Lavandox, Thallasine 2, Argireline NP, Gatuline In-Tense and Gatuline Expression, Myoxinol LS 9736, Synake, and Instensyl^{®}, Sesaflash^{™}, N- acetyl D-glucosamine, panthenol (for example, DL panthenol available from Alps Pharmaceutical Inc.), tocopheryl nicotinate, benzoyl peroxide, 3-hydroxy benzoic acid, flavonoids (for example, flavanone, chaicone), famesol, phytantriol, glycolic acid, lactic acid, 4-hydroxy benzoic acid, acetyl salicylic acid, 2-hydroxybutanoic acid, 2-hydroxypentanoic acid, 2-hydroxyhexanoic acid, cis- retinoic acid, trans-retinoic acid, retinol, retinyl esters (for example, retinyl propionate), phytic acid, N-acetyl-L-cysteine, lipoic acid, tocopherol and its esters (for example, tocopheryl acetate: DL-a- tocopheryl acetate available from Eisai), azelaic acid, arachidonic acid, tetracycline, ibuprofen, naproxen, ketoprofen, hydrocortisone, acetaminophen, resorcinol, hexyl-resorcinol, phenoxyethanol, phenoxypropanol, phenoxyisopropanol, 2,4,4'-trichloro-2'-hydroxy diphenyl ether, 3,4,4'- trichlorocarbanilide, octopirox, lidocaine hydrochloride, clotrimazole, miconazole, ketoconazole, neomycin sulfate, theophylline, and mixtures thereof.

Alternatively, cosmetic agent is a cellulite treatment agent. In some embodiments, the cellulite treatment agent is selected from the group consisting of apigenin, extracts containing Berberine or salts thereof, a blend of pterostilbene (resveratrol dimethyl ether) and xymenynic acid dissolved in glyceryl linoleate and glyceryl linolenate, and combinations thereof.

Cosmetic agent may also be a skin lightening agent, or an antiaging ingredient selected from the group consisting of ascorbic acid compounds, vitamin B3 compounds, azelaic acid, butyl hydroxy anisole, gallic acid and its derivatives, glycyrrhizinic acid, hydroquinone, kojic acid, arbutin, mulberry extract, and combinations thereof. In some embodiments, the topical final formulation may comprise Ovaliss ((S)- 5,6,6a,7-Tetrahydro-l,2,9,10-tetramethoxy-6-methyl-4H-dibenzo[de,g]quinoline, 1,2- Octanediol, D-Glucopyranose, oligomeric, C10-16-alkyl glycosides, water, ethyl alcohol, and glycerin), Whey protein, MPC (Milk protein complex), Sesaflash (Glycerin, Acrylates copolymer, PVP/polycarbamyl polyglycol ester, Hydrolyzed Sesame Protein PG - propyl methylsilanediol), Majestem (glycerin, Leontopodium Alpinum Callus Culture Extract and xanthan gum), Idealift (butylene glycol, sorbitan laurate, hydroxyethylcellulose, and acetyl dipeptide- 1 cetyl ester), and combinations thereof.

The final formulation may further comprise a emollient or lubricating vehicle, emulsifier or surfactant, gelling agent, humectant, sensation modifying agent selected from the group of a cooling agent, a warming agent, a relaxing or soothing agent, a stimulating or refreshing agent, and combinations thereof.

According to a fourth aspect, the present invention is directed to a photosensitive liposomal controlled delivery system, comprising photosensitive liposomes as described above, wherein the release of said one or more active agents is controlled by an exposure to direct UV irradiation, or by the UV-generated radiation sources, chosen among X-ray, and/or near-infrared (NIR) light, promoted by additives like scintillators and/or upconversion materials.

According to this aspect, it is provided the use of the photosensitive liposomes for controlling the release of said one or more active agents, comprising exposing said liposome to direct UV irradiation, to UV-generating radiation sources, thereby allowing photo-induced modification of the said diazirine-conjugated phospholipids and releasing said one or more active ingredients from the liposome.

The present invention also relates to method of delivering one or more active agents in a controlled manner comprising administering one or more active agents encapsulated within liposomes and exposing said liposomes, after administration, to direct UV irradiation, or to UV generated from other radiation sources, chosen among X-ray, and/or near-infrared (NIR) light, promoted by additives like scintillators and/or upconversion materials, thereby allowing releasing said one or more active ingredients from the liposome.

According to this aspect, the present invention is directed to a method of treating and/or preventing a condition responsive to a photosensitive liposome-encapsulated or associated therapeutic agent, comprising the steps of:
(a) administering to a patient a pharmaceutical composition comprising a photosensitive liposomal delivery system comprising one or more therapeutic agent(s), wherein the therapeutic agent is encapsulated in the photosensitive liposomes, and optionally a pharmaceutically acceptable carrier or diluent; and
(b) subjecting the patient to direct UV exposure to destabilize said photosensitive liposomes and to release said one or more therapeutic agent(s) encapsulated in the liposome.

Examples of conditions that are responsive to liposome-encapsulated or associated therapeutic agent(s) include, but are not limited to, cancer, immune disorders, developmental disorders, genetic disorders, and infections. Examples of chemicals that can be encapsulated in or associated with the liposomes described in the present disclosure, include but are not limited to chemotherapeutics, radiopharmaceuticals, radiation sensitizers, contrast agents, fluorescent compounds, genetic materials, biological response modifiers, biological cofactors, cell toxins.

Preferably, it is provided a method of treating and/or preventing cancer in a subject in need thereof comprising (i) administering to the subject an effective dose of the photosensitive liposome comprising at least an anticancer agent, and (ii) exposing the subject to direct UV irradiation, or to UV generated from other radiation sources, chosen among X-ray, and/or near-infrared (NIR) light, promoted by additives like scintillators and/or upconversion materials in order to release said one or more anticancer drugs. Said photosensitive liposomes may be administered via intravenous, subcutaneous, and/or intramuscular routes.

According to a fifth aspect, the present invention provides a process for the preparation of the photosensitive diazirine-conjugated phospholipids, and key intermediates used in the preparation of photosensitive diazirine-conjugated phospholipids. Said key intermediates may be photosensitive lysophospholipids of general formula (KI): 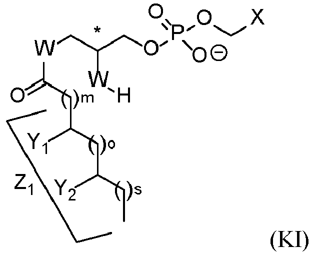
wherein the radical X is chosen among the following groups: 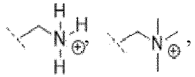 or 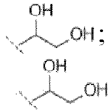
wherein at least one radical Y₁ or Y₂ is a diazirine group: 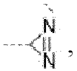 the other radical Y₁ or Y₂ being a hydrogen;
wherein the atom W is O or N;
wherein said phospholipid may have saturated or unsaturated fatty chain Z₁ ;
wherein m, o and s are integers independently ranging from 1 to 8, or from 1 to 7, or from 1 to 6, or from 1 to 5;
wherein the said phospholipid is either achiral or chiral as defined by the absolute configuration of the stereogenic center C^{∗} (R, S or a racemic mixture);
and/or salts thereof.

### EXAMPLES

### Example 1: General synthetic procedures:

The photo-sensitive phospholipids were synthesized from a Lyso-PC precursor and a fatty acid incorporating a diazirine group, as described in the following Scheme.

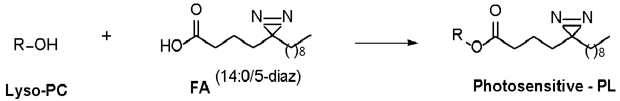

### Example 1.1: Preparation of diazirine-conjugated fatty acid FA (14:0/5-diaz)

The fatty chain incorporating a diazirine group in the C₅ position was synthesized in 4 steps, as showed in the following Scheme. 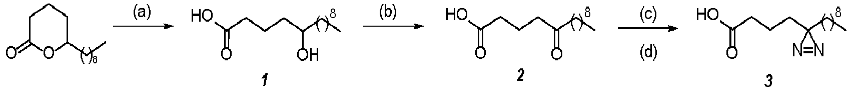 **Scheme**. (a) TMSONa / DCM, 12h, r.t.; (b) NBS / H₂O, EtOAc, 1h, reflux; (c) i. NH₃/MeOH, 3h, 0°C; ii. Hydroxylamine-*O*-sulfonic acid / MeOH, 1h, 0°C the 72h, r.t.; (d) I₂, DIPEA / MeOH, 48h, r.t.

Synthesis of *5-hydroxytetradecanoic acid (**1**).* A solution of TMSONa (3 g, 26.78 mmol, 1.2 eq.) in dry DCM (26.5 ml) was added to a solution of δ-tetradecalactone (5 g, 22.105 mmol, 1 eq.) in dry dichloromethane (12.5 ml). The mixture was stirred at room temperature for 12 h. At the end of the reaction, the solvent was removed under vacuum and the residue was dissolved in DCM (25 ml) and methanol (25 ml). After another cycle of evaporation, the residue was resuspended in DCM (50 ml), and filtered on a glass sintered funnel. The precipitate was dissolved in a MeOH / DCM mixture (50 ml, 1/1), silica (2 g) was subsequently added and the mixture was stirred for 30 min at room temperature. After filtration on a glass sintered funnel, the recovered filtrate was dried under reduced pressure. The obtained solid was subsequently used without further purification. Two batches of the starting material conducted to 9.8 g of the desired alcohol **1. M** = 244.37 g / mol; **¹H NMR** (300 MHz, MeOD) δ 3.53 (Is, 1H), 2.17 (t, 2H, *J* = 7.4 Hz), 1.80 - 1.58 (m, 2H), 1.49 - 1.38 (m, 4H), 1.30 (a Is, 12H), 0.90 (t, *J* = 6.2 Hz, 3H). **MS (ESI):** m/z = 243.2 (M-H), 267.2 (M+Na) ; **IR (ATR)** : 3337, 2918, 1557, 1413, 1097, 845; **Mₚ** = 140.6°C.

Synthesis of *5-oxotetradecanoic acid (**2**). N*-bromosuccinimide (21.4 g, 120.24 mmol, 3eq.) was added to a solution of the previously obtained crude alcohol 1 (9.8 g, 40 mmol, 1 eq.) in a water/ethyl acetate mixture (250 ml / 350 ml). The reaction was refluxed for 1 h and subsequently cooled to room temperature. Sodium thiosulfate was added until the complete disappearance of the light brown color and the organic materials were extracted with ethyl acetate (3 × 300 ml). The combined organic phases were dried over magnesium sulfate, filtered and the solvent was evaporated under reduced pressure.

The crude product was purified by flash chromatography (DCM/MeOH 99/1) to give a white solid that was subsequently recrystallized in ethanol to conduct to the final compound **2** as a white solid (5.8 g). **M** = 242.40 g / mol; **Yield** (59% over two steps); **¹H NMR** (300 MHz, MeOD) δ 2.52 (t, 2H, *J* = 7.2 Hz), 2.44 (t, 2H, *J* = 7.4 Hz), 2.30 (t, 2H, *J* = 7.3 Hz), 1.82 (p, 2H, *J* = 7.3 Hz), 1.5-1.47 (m, 2H), 1.29 (a Is, 12H), 0.90 (t, 3H, *J* = 7.0 Hz); **MSI (ESI):** m/z = 265.2 (M+Na); **IR (ATR):** 3110, 2915, 1695, 1418, 1289, 1099 cm⁻¹; **M**ₚ = 77 - 79°C.

Synthesis of *4-(3-nonyl-3H-diazirin-3-yl)butanoic acid (**3**).* The previously obtained ketone **2** (4 g, 16.5 mmol, 1 eq.) was dissolved in ammonia (7N in methanol, 45 ml) and the reaction was stirred at 0°C for 3 h. Hydroxylamine-*O*-sulfonic acid (2.42 g, 21.47 mmol, 1.3 eq.) in dry methanol (14 ml) was subsequently added to the mixture, and the reaction was kept under stirring for an additional hour at 0°C, then for 72 h at room temperature. At the end of the reaction, nitrogen gas was bubbled into the reaction mixture, the precipitate was filtered and the filtrate was evaporated under reduced pressure. The obtained crude was subsequently used without further purification for the next step. The crude diaziridine (4.24 g, 16.53 mmol, 1 eq.) was dissolved in methanol (40 ml) and *N,N-*diisopropylethylamine (2.5 ml) was added to the reaction mixture. After 5 minutes of stirring, solid iodine was slowly added to the reaction until a persistence of the brown color. The reaction was continued for 48 h at room temperature before the addition of ethyl acetate (40 ml) and of hydrochloric acid (to a final pH = 3 of the aqueous layer). Sodium thiosulfate was subsequently added to the mixture until the complete disappearance of the brown color. The organic materials were extracted with ethyl acetate (3 × 50 ml), the combined organic layers were dried over magnesium sulfate, filtered and the solvent was evaporated under reduced pressure. The obtained crude was purified by flash-chromatography (DCM/MeOH 99/1) to conduct to **3** as a white gum (1.98 g). **M** = 254.37 g/mol; **Yield** (47 % over two steps); **¹H NMR** (300 MHz, CDCl₃) δ 2.31 (t, *J* = 7.0 Hz, 2H), 1.48 - 1.40 (m, 4H), 1.37 - 1.35 (m, 2H), 1.30 - 1.16 (m, 12H), 1.08 - 1.05 (m, 2H), 0.87 (t, 3H, J = 7.0 Hz); **MSI (ESI)** : m/z = 253.19 (M-H); **IR (ATR)** : 1918, 1690, **1574,** 1438, 1286, 1221, 913 cm⁻¹.

### Example 1.2: Preparation of diazirine-conjugated phospholipids (photosensitive-PLs)

The photo-sensitive phospholipids were synthesized from a Lyso-PC precursor and a carboxylic acid incorporating a diazirine group in C₅ using a coupling procedure, as described in the following Scheme.

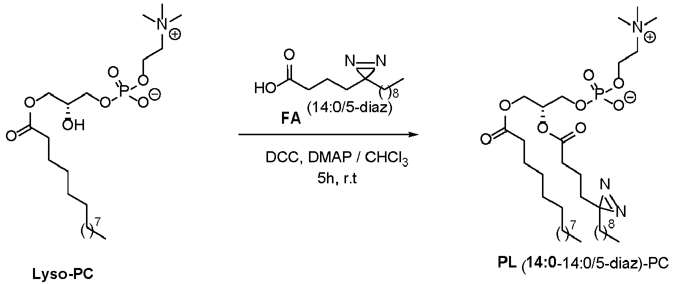

### Example 2: Preparation of diazirine-conjugated phospholipid of formula (III)

Synthesis of *(R)-2-((4-(3-nonyl-3H-diazirin-3-yl)butanoyl)oxy)-3-(tetradecanoyloxy)propyl (2-(trimethylammonio)ethyl)phosphate.* Under inert atmosphere, a solution of the previously obtained acid **3** (0.3 g, 1.17 mmol, 3 eq.), DCC (0.241 g, 1.17 mmol, 3 eq.), DMAP (0.142 g, 1.17 mmol, 3 eq.), Lyso-PC (14:0) (0.183 g, 0.39 mmol, 1 eq.) in dry chloroform (12 ml) was sonicated in an ultrasound bath at room temperature in the presence of glass beads (0.45 g) for 5 h. After the complete consumption of the starting material, the reaction mixture was directly purified by flash chromatography (chloroform / methanol / water 65/ 25 / 4) to conduct to the desired photo-sensitive phospholipid **III** (EB168, 0.203 g) as a white hygroscopic solid. **M** = 703.94 g/mol; **Yield** : 74%; **¹H NMR** (300 MHz, CDCl₃) δ 5.23 - 5.16 (m, 1H), 4.59 (sl, 1H), 4.40 (dd, 1H, *J₁* = 3.1 Hz, *J₂* = 12.0 Hz), 4.25 - 4 .20 (m, 2H), 4.13 (dd, 1H, *J₁* = 6.9 Hz, *J₂* = 12.0 Hz), 3.96 (t, 2H, *J* = 6.1 Hz), 3.63 - 3.60 (m, 2H), 3.20 (s, 9H), 2.29 (t, 4H, *J* = 7.4 Hz), 1.59 - 1.55 (m, 2H), 1.40 - 1.32 (m, 6H), 1.26 (a sl, 30H), 1.07-1.03 (m, 2H), 0.86 (t, 6H, *J* = 6.6 Hz); **MSI (ESI)** : m/z = 704.2 (M+H), 726.5 (M+Na); **IR (ATR)** : 3391, 2920, 1734, 1579, 1468, 1236, 1062, 966, 794 cm⁻¹.

### Example 3: Preparation of diazirine-conjugated phospholipid formula (IV)

Synthesis of (R)-2-((4-(3-nonyl-3H-diazirin-3-yl)butanoyl)oxy)-3-(palmitoyloxy)propyl (2-(trimethylammonio)ethyl) phosphate. Under inert atmosphere, a solution of the previously obtained acid 3 (0.2 g, 0.787 mmol, 3 eq.), DCC (0.162 g, 0.787 mmol, 3 eq.), DMAP (0.096 g, 0.787 mmol, 3 eq.), Lyso-PC (16:0) (0.129 g, 0.262 mmol, 1 eq.) in dry chloroform (8 ml) was sonicated in an ultrasound bath at room temperature in the presence of glass beads (0.3 g) for 5 h. After the complete consumption of the starting material, the reaction mixture was directly purified by flash chromatography (chloroform / methanol / water 65 / 25 / 4) to conduct to the desired photo-sensitive phospholipid **IV** (EB177, 0.135 g) as a white hygroscopic solid. M = 732.0 g/mol; Yield (76%); **¹H NMR** (300 MHz, CDCl₃) δ 5.23 - 5.11 (m, 1H), 4.37 (dd, 1H, *J₁* = 2.6 Hz, *J₂* = 12.0 Hz), 4.28 (a Is, 2H), 4.09 (dd, 1H, *J₁* = 7.2 Hz, *J₂* =12.0 Hz), 3.95 - 3.65 (m, 7H), 3.35 (s, 9H), 2.26 (a t, 4H, *J* = 7.5 Hz), 1.65 - 1.49 58 (m, 2H), 1.43 - 1.12 (m, 42H), 1.11 - 0.94 (m, 2H), 0.86 (t, 6H, *J* = 6.4 Hz). **MSI (ESI):** m/z = 732.5 (M+H), 754.5 (M+Na); **IR (ATR)** : 3389, 2919, 1735, 1467, 1240, 1086, 968, 798.

### Example 4: Preparation of diazirine-conjugated phospholipid (photosensitive-PL) of formula (V)

Synthesis of (R)-2-((4-(3-nonyl-3H-diazirin-3-yl)butanoyl)oxy)-3-(stearoyloxy)propyl (2-(trimethylammonio)ethyl) phosphate. Under inert atmosphere, a solution of the previously obtained acid **3** (0.42 g, 1.639 mmol, 3 eq.), DCC (0.338 g, 1.639 mmol, 3 eq.), DMAP (0.200 g, 1.639 mmol, 3 eq.), Lyso-PC (18:0) (0.286 g, 0.546 mmol, 1 eq.) in dry chloroform (16 ml) was sonicated in an ultrasound bath at room temperature in the presence of glass beads (0.6 g) for 7.5 h. After the complete consumption of the starting material, the reaction mixture was directly purified by flash chromatography (chloroform / methanol / water 65 / 25 / 4) to conduct to the desired photo-sensitive phospholipid **V** (EB167, 0.3356 g) as a white hygroscopic solid. **M** = 760.3 g/mol; **Yield** : 81%; **¹H NMR** (600 MHz, CDCl₃) δ 5.18 - 5.15 (m, 1H), 4.37 (dd, 1H, *J₁* = 2.6 Hz, *J₂* = 12.0 Hz), 4.28 (a Is, 2H), 4.10 (dd, 1H, *J₁* = 7.2 Hz, *J₂* = 12.0 Hz), 3.95 - 3.87 (m, 2H), 3.78 (a Is, 2H), 3.35 (s, 9H), 2.28 - 2.24 (m, 4H), 1.58 - 1.55 (m, 2H), 1.41 - 1.38 (m, 4H), 1.35 (t, 2H, *J* = 7.8 Hz), 1.27 - 1.21 (m, 40H), 1.06 - 1.02 (m, 2H), 0.86 (t, 6H, *J* = 6.4 Hz); **MSI (ESI)** : m/z = 760.5 (M+H), 782.5 (M+Na); **IR (ATR)** : 3377, 2919, 1731, 1579, 1467, 1250, 1053, 966, 817 cm⁻¹;

### Example 5: Pharmaceutical composition or kit comprising the photosensitive liposomal delivery system

### Example 5.1: Liposome preparation

Small unilamellar vesicles (SUVs) were prepared by the thin film hydration method. Briefly, the phospholipids (1 mg) were dissolved in a chloroform (0.2 ml) in a glass test tube. The organic solvent was gently removed by evaporation using a N₂ flux and the samples were further dried under high vacuum for at least 1 hour. The resulting thin lipid films were hydrated for 10 mins at 35°C with 100 mM calcein (0.5 ml) in phosphate buffer saline solution (10 mM phosphate buffer, 154 mM sodium chloride, pH = 7.4) and vortexed for 3 minutes. The obtained 2 mg/ml liposomal suspensions were subjected to five cycles of freezing and thawing to obtain unilamellar vesicles: freezing 3 minutes using liquid nitrogen and thawing 3 minutes at 50°C. The obtained solutions were extruded through polycarbonate filter (100 nm pore size filter, 21 times) using Avanti^{®} extruder on a thermal plate maintained at 35°C. A control sample consisting in DMPC liposomes was prepared using the same method as described above.

The size and polydispersity of the samples were assessed by Dynamic Light Scattering (Malvern, Zetasizer Pro), and their morphology validated by Transmission Electron Microscopy (Talos F200S G2, ThermoFisher).

The free un-encapsulated calcein was removed by SEC Sephadex G75 (1.6 cm × 20 cm) column. The fractions (0.5 ml) were collected and DLS and fluorescence were used to assess the quality of separation and relative liposomal concentrations. The SEC fraction concentrated with liposomes was used for further investigations.

The total release of encapsulated calcein was achieved by sonication/vortexing cycles and monitored by fluorescence measurement, as follows: 90 µl of the liposome concentrated fraction was mixed with 810 µl of PBS and subjected to cycles of 10 minutes of sonication and 3 minutes of vortexing. This was repeated until a maximum fluorescence was attained after around 3 to 4 cycles.

### Example 6: UV Triggered Release of Liposomal Content

Liposomes comprising a photo-sensitive lipid component of the present disclosure were investigated for their ability to release an encapsulated a fluorophore dye mimicking a hydrophilic therapeutic agent, upon exposure to UV light.

Samples (1 mg phospholipids in 0.5 ml of a solution of 100 mM calcein in PBS 1×), prepared as described above, were irradiated for 60 minutes in a 2 ml glass vial, at a distance of 1 cm using a UV lamp at 365 nm. The UV-light dose received by the sample was 4 mW. After exposure, the encapsulated calcein was separated from the free calcein by SEC as mentioned above. Fractions of 0.5 ml were collected. 30 µl of the most concentrated fraction in liposomes (as assessed by DLS) was mixed with 2.97 ml PBS and transferred for calcein release analysis by recording the emission fluorescence at different time intervals: initial 10, 30, 60 mins then every 1 hour until five hours. The fluorescence signal was normalized to 1 (100%) by considering the magnitude of the fluorescence signal recorded upon total release achieved by sonicate/vortex cycles. The release efficiency (% Release) was calculated by considering the fluorescence of the sample before irradiation (Fo), the fluorescence of the sample after exposure and at different time intervals (Fₓ), and the maximal fluorescence (Fₜₒₜₐₗ). The following formula was used: % Release = 100 × (Fₓ - F₀) /(Fₜₒₜₐₗ - F₀). The "Normalized % Release" was calculated taking into account that the maximum "% Release" by passive diffusion is 50%.

Dye-loaded liposomes obtained from EB168 phospholipids (synthesized as described before) and control phospholipids (DMPC) were prepared as summarized before. They showed similar sizes: between 120-140 nm by DLS - Figure 1A. Separation of the calcein-loaded liposomes from the free dye showed that, contrarily to the control liposomes which were unable to incorporate the fluorescent dye (Figure ID), the photosensitive liposomes were able to encapsulate and store their load during the size-extrusion chromatography phase (fractions F₇-F₉ - Figure 1C). The dye leaking *via* a passive diffusion process was monitored for the irradiated EB168 samples in comparison to the non-irradiated EB168 samples. As showed in Figure 1B, no leaking was observed for the non-irradiated samples during the first 5 hours of monitoring, while for the irradiated samples the dye escape attended 31% of the maximum release that can be reached by passive diffusion. In conclusion, irradiation of the photo-sensitive liposomes triggered morphological transformations of the liposomes, which induced liposomes leakage. These morphological modifications are the cumulative effects of: (1) liposomal shrinkage induced by photo-crosslinking reactions between the phospholipids; (2) photo-oxidation products of the photo-sensitive phospholipids with O₂ and water; (3) photo-reduced products generated from the photo-sensitive phospholipids.

## Claims

1. Photosensitive diazirine-conjugated phospholipid according to claim 1, wherein W=O and having the following general formula (II): 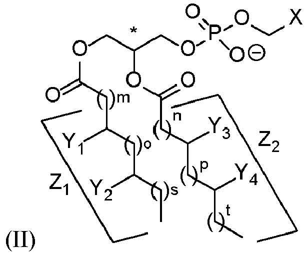
wherein the radical X is chosen among the following groups: 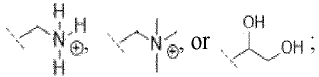
wherein at least one radical Y₁, Y₂, Y₃ or Y₄ is a diazirine group : 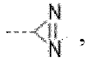 the other radical Y₁, Y₂, Y₃ or Y₄ being a hydrogen;
wherein said phospholipids are saturated or unsaturated fatty chains Z₁ or Z₂;
wherein m, n, o, p, s, t are integers independently equal to 1 to 8, or 1 to 7, or 1 to 6, or 1 to 5; and
wherein the said phospholipid is either achiral or chiral as defined by the absolute configuration of the stereogenic center C^{∗} (R, S or a racemic mixture);
and/or salts thereof.

2. Photosensitive diazirine-conjugated phospholipid according to claim 1, wherein said phospholipid is a natural or synthetic phospholipid, and/or wherein said phospholipid is natural and is chosen among phosphatidylcholine, phosphatidylserine, phosphatidylinositol, phosphatidic acid, phosphatidylglycerol, or phosphatidylethanolamine.

3. A photosensitive liposome comprising one or more photosensitive diazirine-conjugated phospholipids according to claim 1 or 2.

4. Photosensitive liposome comprising one or more photosensitive diazirine-conjugated phospholipids according to any one of claims 1-3 and optionally non-diazirine conjugated phospholipids, wherein the proportion of said diazirine-conjugated phospholipids is between 5-100%.

5. Photosensitive liposome according to claim 3 or 4, wherein the photosensitive group is a diazirine, and wherein modification of said diazirine is photo-induced.

6. Photosensitive liposome according to claim 6, wherein photo-induced modification of the diazirine is activated by an exposure to direct UV irradiation, or to UV-generated radiation sources.

7. Photosensitive liposome according to claim 7, wherein said UV-generating radiation sources are chosen among scintillators and/or upconversion materials, and wherein scintillators generate UV when exposed to X-ray, and upconversion materials generate UV when exposed to or near-infrared (NIR) light.

8. Photosensitive liposome according to claim 7, wherein said scintillators and/or upconversion materials which respectively generate UV when exposed to X-ray, and/or near-infrared (NIR) light are nanomaterials or lanthanides chosen among lanthanum, cerium, ytterbium, yttrium, erbium, thulium, their salts and complexes.

9. Photosensitive liposome according to any one of claims 3-8, wherein one or more active agents are encapsulated within said liposome.

10. Photosensitive liposome according to claim 9, wherein said one or more active agents are hydrophilic, and are chosen among drugs, anticancer drugs, enzymes, peptides, proteins, toxins, nucleic acids, chemotherapeutics, pharmaceuticals, cosmetic agents, antifungal agents, radiopharmaceuticals, radiation sensitizers, contrast agents, iodinated agents, fluorescent compounds, and/or a combination thereof, or wherein a targeting molecule is present on the external surface of the liposome.

11. A photosensitive liposomal controlled delivery system comprising liposome as defined in any one of claims 3-10, wherein the release of said one or more active agents is controlled by an exposure to direct UV irradiation, or by the UV-generated radiation sources, chosen among X-ray, and/or near-infrared (NIR) light, promoted by additives like scintillators and/or upconversion materials.

12. The photosensitive liposomal controlled delivery system of claim 11, wherein said scintillators and/or upconversion materials which generate UV when rexposed to X-ray, and/or near-infrared (NIR) light, respectively, are nanomaterials or lanthanides chosen among lanthanum, cerium, ytterbium, yttrium, erbium, thulium, their salts and complexes

13. A composition or kit comprising the photosensitive liposomal delivery system according to claim 11 or 12, and wherein said one or more active agents are a therapeutically active agent, or a diagnostic agent, or a cosmetic agent.

14. A method of delivering one or more active agents in a controlled manner comprising administering one or more active agents encapsulated within liposomes as defined in any one of claims 9-13 and exposing said liposomes, after administration, to direct UV irradiation, or to UV generated from other radiation sources, chosen among X-ray, and/or near-infrared (NIR) light, promoted by additives like scintillators and/or upconversion materials, thereby allowing releasing said one or more active ingredients from the liposome.

15. Intermediates for the preparation of the photosensitive diazirine-conjugated phospholipid according to claim 1 or 2 having the following general formula (KI): 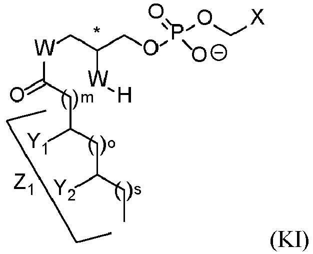
wherein the radical X is chosen among the following groups: 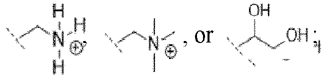
wherein at least one radical Y₁ or Y₂ is a diazirine group: 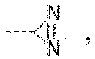 the other radical Y₁ or Y₂ being a hydrogen;
wherein the atom W is O or N;
wherein said phospholipid may have saturated or unsaturated fatty chain Z₁ ;
wherein m, o and s are integers independently equal to 1 to 8, or 1 to 7, or 1 to 6, or 1 to 5;
wherein the said phospholipid is either achiral or chiral as defined by the absolute configuration of the stereogenic center C^{∗} (R, S or a racemic mixture);
and/or salts thereof.
